# EUROPEAN PATENT APPLICATION

(11) **EP 4 806 350 A1**
(43) Date of publication of application: **16.09.2026**
(21) Application number: 26163544.5
(22) Date of filing: 10.03.2026
(51) Int. Cl.: A61B 5/145

(54) **IMPLANTATION DEVICE FOR IMPLANTING ANALYTE SENSOR INTO SUBCUTANEOUS TISSUE**

(30) Priority: 12.03.2025 CN 202510292618; 12.03.2025 CN 202510292619; 12.03.2025 CN 202520433019 U
(71) Applicant: Jiangsu Yuwell POCTech Biotechnology Co., Ltd., Zhenjiang, Jiangsu 212300 (CN); Jiangsu Yuekai Biotechnology Co., Ltd., Nanjing, Jiangsu 210018 (CN); Zhejiang Poctech Co., Ltd., Huzhou, Zhejiang 313001 (CN)
(72) Inventor: ZHANG, Yanan, Nanjing, Jiangsu, 210018 (CN); McCANLESS, Jonathan, Nanjing, Jiangsu, 210018 (CN); WANG, Liang, Nanjing, Jiangsu, 210018 (CN); ZHU, Jiandong, Nanjing, Jiangsu, 210018 (CN)
(74) Representative: Lorenz Seidler Gossel Part. mbB

(57) **Abstract**

This application discloses an implantation device for implanting an analyte sensor into a subcutaneous tissue, including an outer housing having a first end and a second end that are oppositely disposed in a first direction, where a mounting port is formed in the first end, and a mounting channel is disposed within the outer housing; a sealing unit disposed within the mounting channel and having a sealed chamber; a monitoring unit including a housing, an electronic module, and a monitoring module, where the housing is arranged at the first end; the electronic module is disposed within the housing; the monitoring module is disposed within the sealed chamber; and a driving unit capable of driving the monitoring module to move in the first direction such that the monitoring module is assembled to the housing, the monitoring module is electrically connected to the electronic module, and the monitoring module is partially implanted into a subcutaneous tissue of an implantee. In this application, the independent sealing unit is provided inside the outer housing, and the monitoring module is mounted inside the sealing unit to form a sterilization unit, so that a volume of the sterilization unit can be reduced, and a plurality of sterilization units can be sterilized once, thereby enhancing sterilization efficiency and reducing sterilization costs.

## Description

### TECHNICAL FIELD

This application belongs to the technical field of medical devices, and specifically, to an implantation device for implanting an analyte sensor into a subcutaneous tissue.

### BACKGROUND

Detecting various analytes in the body of an individual is crucial for monitoring a health condition of the individual, and a deviation from a normal analyte level can usually indicate a potential physiological condition, such as a metabolic condition or a disease. Periodically performing in-vitro analyte monitoring by using an extracted body liquid is sufficient to observe physiological conditions of many individuals. However, due to a limited quantity of detection values in the in vitro analyte monitoring, it is possible to miss an optimal treatment timing due to lack of key measurement data and cause irreversible injury to a patient. In addition, for an individual with a serious imbalance of an analyte and/or a rapid fluctuation in the level of the analyte, a body liquid relatively needs to be frequently extracted for monitoring. This causes inconvenience and pains to the patient.

In many cases, subcutaneous, interstitial, or skin analyte sensors can provide sufficient measurement precision and reduce the discomfort of a user to the greatest extent. Continuous analyte monitoring by using an analyte monitoring sensor implanted into the body is an ideal monitoring method. Generally, an implantation device is used to implant an analyte monitoring sensor into the body of an individual. The sensor reacts with a body liquid of implantee to generate an electrical signal. A processing unit converts the electrical signal into data that can represent an analyte concentration and then transmits the data to a display device for displaying, thereby implementing continuous monitoring on the analyte concentration.

At each time a new sensor is used, a user implants part of the sensor under the skin of the user. Usually, the implantation device is used to insert the sensor into the user. A puncture needle inside the implantation device is connected to the sensor and brings the sensor under the skin of the user. After the sensor is implanted in place, the puncture needle is removed from the body of the user, and the sensor is maintained in the body of the user. Before use, both the puncture needle and the sensor need to be sterilized. The existing implantation device has relatively low sterilization efficiency, high sterilization costs, and complex assembling process. Before use, a user needs to perform operations such as unpacking and removing a component that is unrelated to an implantation process. Many operation steps will reduce a user experience and increase a probability of a sensor implantation failure caused by an improper user operation.

### SUMMARY

This application provides an implantation device for implanting an analyte sensor into a subcutaneous tissue, which can enhance sterilization efficiency, reduce sterilization costs, and simplify a manufacturing process. It can also improve the user experience and reduce the probability of implantation failure, thereby addressing the problems associated with existing implantation devices, such as inconvenient gripping, relatively high cost, and susceptibility to operational errors. The implantation device includes:
an outer housing, where the outer housing has a first end and a second end that are oppositely disposed in a first direction; a mounting port is formed in the first end, and a mounting channel extending in the first direction is provided inside the outer housing;
a sealing unit disposed within the mounting channel, where the sealing unit has a sealed chamber, wherein the sealing chamber and the mounting channel are isolated from each other;
a monitoring unit, where the monitoring unit includes a housing, an electronic module, and a monitoring module; the housing is detachably arranged at the first end; the electronic module is disposed within the housing; the monitoring module is disposed within the sealed chamber; and
a driving unit, where the driving unit is disposed within the mounting channel; the driving unit is capable of driving the monitoring module to move in the first direction such that the monitoring module is assembled to the housing, the monitoring module is electrically connected to the electronic module, and the monitoring module is partially implanted into a subcutaneous tissue of an implantee.

In one possible implementation, the driving unit is located outside the sealed chamber, and the driving unit is capable of entering the sealed chamber in the first direction to push the monitoring module to move.

In one possible implementation, the sealing unit is provided with communication openings respectively in two ends in the first direction, and the communication openings are configured to communicate the sealed chamber with the mounting channel.

In one possible implementation, the sealing unit includes a receiving member and a sealing member detachably connected to the receiving member; the receiving member and the sealing member enclose the sealed chamber; the communication openings include a first communication opening and a second communication opening that are located at two opposite ends of the receiving member; the sealing member includes a first sealing portion and a second sealing portion; the first sealing portion seals the first communication opening; and the second sealing portion seals the second communication opening.

In one possible implementation, the sealing member further includes a first folding portion and a second folding portion that respectively extend from the first sealing portion and the second sealing portion, and a first operation portion and a second operation portion that respectively extend from the first folding portion and the second folding portion; one side of the first folding portion facing away from the sealed chamber and one side of the second folding portion facing away from the sealed chamber are respectively stacked on the first sealing portion and the second sealing portion; a withdrawal port is provided in the outer housing; and the first operation portion and the second operation portion extend out of the outer housing from the withdrawal port.

In one possible implementation, the implantation device further includes a bottom housing detachably fixed to the first end; the first operation portion and the second operation portion are fixed to the bottom housing; and the bottom housing is separated from the outer housing to separate the sealing member from the receiving member.

In one possible implementation, a fixing member is further disposed within the outer housing; the fixing member is located between the sealing unit and the second end; the sealing unit is fixedly connected to the fixing member; the fixing member has an opening corresponding to the communication openings; and the driving unit is disposed between the fixing member and the second end.

In one possible implementation, the sealing unit is provided with a clamping hook, and the clamping hook passes through the opening and is clamped and fixed to the fixing member.

In one possible implementation, the housing has a mounting position for accommodating the monitoring module; the monitoring module is capable of moving in the first direction under driving of the driving unit, so as to be mounted in the mounting position; and a puncture unit penetrates the subcutaneous tissue.

In one possible implementation, the monitoring module is provided with a first clamping portion; a second clamping portion is disposed within the mounting position; the first clamping portion and the second clamping portion are clamped and fixed; a first electrical connection portion is provided inside the mounting position; the monitoring module is provided with a second electrical connection portion; and when the monitoring module is placed into the mounting position, the first electrical connection portion is electrically connected to the second electrical connection portion.

In one possible implementation, the outer housing is provided with an elastic arm extending into the mounting port and a fixed portion arranged at an end of the elastic arm; and the fixed portion is abutted with an outer circumferential surface of the housing.

In one possible implementation, a contact member is arranged on one side of the fixed portion facing the housing; and the contact member is made of a flexible material and is elastically abutted with the housing.

In one possible implementation, the implantation device further comprises a bottom housing, wherein the bottom housing is detachably connected to the outer housing, wherein the sealed chamber communicates with the mounting channel when the bottom housing is separated from the outer housing.

In one possible implementation, the implantation device further comprises a driving unit and a puncture unit; at an initial position, the driving unit and the puncture unit are both disposed within the mounting channel and are located outside the sealed chamber; and the driving unit is able to drive the puncture unit to move in the first direction, so that the puncture unit and the monitoring unit are assembled and the puncture unit and the monitoring unit at least partially penetrate the subcutaneous tissue.

In one possible implementation, the implantation device further comprises a puncture unit; the puncture unit comprises a hub and a puncture needle connected to the hub; the driving unit comprises a driving member and a connector connected between the driving member and the hub; at an initial position, the hub is located inside the sealed chamber and is fixedly connected to the monitoring unit; and the connector is located outside the sealed chamber.

In one possible implementation, the connector is provided with a clamping structure; the hub is provided with a fitting structure; and the connector is able to move in the first direction to connect the clamping structure to the fitting structure.

In one possible implementation, the driving unit is provided with a stop structure; the puncture unit is provided with a fitting portion; the fitting portion fits with the stop structure to prevent the puncture unit from moving relative to the driving unit; an unlocking structure is disposed within the mounting channel; and the unlocking structure is able to apply a force to the fitting portion or the stop structure to detach the fitting portion from the stop structure.

In one possible implementation, the puncture unit is connected to the driving unit; an unlocking structure is arranged at an edge of the opening; the unlocking structure is able to be in abutment fit with the puncture unit to unlock the puncture unit, so that the puncture unit moves relative to the driving unit in a second direction; and the second direction is opposite to the first direction.

In one possible implementation, the monitoring unit comprises a housing, an electronic module, and a monitoring module; the housing is detachably arranged at the first end; the electronic module is disposed within the housing; the monitoring module is disposed within the sealed chamber; the monitoring module is able to move in the first direction under driving of the driving unit, so that the monitoring module is assembled to the housing, and the monitoring module is partially implanted into a subcutaneous tissue of an implantee.

In one possible implementation, the monitoring unit comprises a housing, an adhesive member arranged on a bottom surface of the housing, and an isolation membrane separable from the adhesive member; and when the bottom housing is separated from the outer housing, the isolation membrane is separated from the adhesive member.

In one possible implementation, an outer diameter of the housing is less than an inner diameter of the mounting port, and the inner diameter of the mounting port is less than or equal to an outer diameter of the adhesive member.

In one possible implementation, a ratio of an outer diameter of the outer housing to a length of the outer housing in the first direction is 1: 3 to 1: 6.

In one possible implementation, the outer housing has an abutment section located at an end of the outer housing in the first direction; the mounting port is located at the abutment section; the abutment section is configured to be abutted with the skin of an implantee; and an outer diameter of the abutment section is 28 mm to 35 mm.

In one possible implementation, an outer diameter of the outer housing is 28 mm to 35 mm.

In one possible implementation, a length of the outer housing is 100 mm to 120 mm.

In one possible implementation, the driving unit comprises a driving member; the mounting channel has a fitting end facing away from the mounting port in the first direction; a boosting spring is disposed between the fitting end and the driving member; a limiting slot is formed in the fitting end and/or the driving member; and an end portion of the boosting spring is disposed within the limiting slot.

In one possible implementation, the unlocking member is disposed on the outer circumferential surface of the outer housing and is able to move in the first direction or the second direction to unlock the driving unit.

In one possible implementation, the unlocking member comprises a manipulation portion located outside the outer housing, a trigger portion located inside the mounting channel, and a connection rib connected between the trigger portion and the manipulation portion; the manipulation portion is configured as the slide button; an avoidance slot through which the connection rib passes is formed in the outer housing; and the avoidance slot extends in the first direction.

In one possible implementation, a receiving slot extending in the first direction is formed in an outer surface of the outer housing; and the slide button is located inside the receiving slot.

In one possible implementation, the driving unit comprises a stop portion; the outer housing comprises a stopper fitting with the stop portion; the stop portion fits with the stopper to prevent the driving unit from moving relative to the outer housing; and the trigger portion is able to extend from the avoidance slot into the mounting channel and be abutted with the stop portion or the stopper to detach the stop portion from the stopper.

In one possible implementation, the trigger portion has a mounting slot; the stop portion passes through the mounting slot to be in abutment fit with the stopper; the mounting slot and/or the stop portion are/is provided with a guide slope; and the trigger portion drives the stop portion through the guide slope, so that the stop portion is detached from the stopper.

In one possible implementation, the monitoring unit further comprises a monitoring module and an electronic module; the electronic module is disposed within the housing; and the monitoring module and the housing are spaced apart in the first direction.

In one possible implementation, the housing has a mounting position for receiving the monitoring module; and a trigger member is triggered to mount the monitoring module into the mounting position and cause the puncture unit to penetrate the subcutaneous tissue.

In one possible implementation, the monitoring module is provided with a first clamping portion; a second clamping portion is disposed within the mounting position; and the first clamping portion and the second clamping portion are clamped and fixed.

In one possible implementation, a first electrical connection portion is provided inside the mounting position; the monitoring module is provided with a second electrical connection portion; and when the monitoring module is placed into the mounting position, the first electrical connection portion is electrically connected to the second electrical connection portion.

In one possible implementation, the outer housing is provided with an elastic arm extending into the mounting port and a fixed portion arranged at an end of the elastic arm; and the fixed portion is abutted with an outer circumferential surface of the housing.

In one possible implementation, a contact member is arranged on one side of the fixed portion facing the housing; and the contact member is made of a flexible material and is elastically abutted with the housing.

In one possible implementation, the implantation device further comprises a bottom housing detachably fixed to the mounting port; the bottom housing closes the mounting port; the bottom housing has a supporting portion protruding in the second direction; and the supporting portion is abutted with a bottom surface of the monitoring unit to support the monitoring unit.

In one possible implementation, the bottom housing comprises a connection section connected to the outer housing and a supporting section formed by extending outwards from the connection section in a radial direction; and an outer contour of the supporting section is arc-shaped.

In one possible implementation, a clamping convex rib is arranged on one of an outer wall of the outer housing and an inner wall of the connection section, and a clamping slot is formed in the other one of the outer wall of the outer housing and the inner wall of the connection section; and the clamping convex rib fits with the clamping slot to fix the bottom housing to the outer housing.

By using the above technical solutions, this application have the beneficial effects:
In this application, the independent sealing unit is provided inside the outer housing, and the monitoring module is mounted inside the sealing unit to form an independent sterilization unit, so that a volume of the sterilization unit that requires irradiation sterilization can be reduced, and a plurality of sterilization units can be sterilized once, thereby enhancing sterilization efficiency and reducing sterilization costs. In addition, the minimization of the sterilization unit helps to minimize the entire implantation device, so that the costs can be reduced. Moreover, the sterilization unit is an independent unit, so that it is relatively convenient to assemble the sterilization unit, the assembling process can be optimized, and a degree of automation can be increased. The bottom housing of the present application is detachably connected to the outer housing. When the bottom housing is separated from the outer housing, the sealing chamber communicates with the mounting channel, such that the bottom housing can be removed and the seal released in a single operation. This reduces the number of steps required from the user and improves the user experience. In addition, it can effectively prevent situations in which the user forgets to release the seal before starting implantation, thereby reducing the probability of implantation failure. In the present application, the unlocking member is a small-sized sliding button located on one side of the outer housing. This configuration can effectively reduce the radial dimension of the outer housing, thereby enabling a miniaturized design, and also allows the user to trigger the device in a more comfortable manner. For example, the user may rotate the outer housing to adjust the orientation of the control portion, thereby completing the triggering action with a more familiar finger and at a more comfortable angle, which improves the user experience. At the same time, it can effectively prevent accidental triggering by the user, thereby avoiding scrapping of the implantation device due to user misoperation.

### BRIEF DESCRIPTION OF THE DRAWINGS

The accompanying drawings described herein are used to provide a further understanding of this application, and form part of this application. Exemplary embodiments of this application and descriptions thereof are used to explain this application, and do not constitute any inappropriate limitation to this application. In the accompanying drawings:
FIG. 1 is a schematic diagram of a structure of an implantation device according to an implementation of this application;
FIG. 2 is a cross-sectional view of the implantation device in FIG. 1;
FIG. 3 is a schematic diagram of a structure of an outer housing according to an implementation of this application;
FIG. 4 is a cross-sectional view of an implantation device according to an implementation of this application, where a bottom housing is not shown;
FIG. 5 is a cross-sectional view of an implantation device in another viewing angle according to an implementation of this application;
FIG. 6 is a cross-sectional view of an implantation device in an initial state according to an implementation of this application;
FIG. 7 is a cross-sectional view of the implantation device in FIG. 6 in a completely implanted state;
FIG. 8 is a schematic diagram of a partial structure of an implantation device according to an implementation of this application;
FIG. 9 is a schematic diagram of a partial structure of an implantation device in another viewing angle according to an implementation of this application;
FIG. 10 is a cross-sectional view of a sealing unit according to an implementation of this application;
FIG. 11 is a schematic diagram of a structure of a sealing unit in FIG. 10;
FIG. 12 is a schematic diagram of a structure of a monitoring module according to an implementation of this application;
FIG. 13 is a schematic diagram of a structure of a bottom housing according to an implementation of this application;
FIG. 14 is a schematic diagram of a structure of a fixing ring according to an implementation of this application; and
FIG. 15 is a schematic diagram of a structure of a housing according to an implementation of this application.
Where:
1: outer housing; 11: mounting channel; 111: stopper; 12: receiving slot; 121: clearance slot; 13: fitting end; 14: limiting slot; 15: guide protrusion strip; 16: abutment section; 17: fixing ring; 171: positioning rib; 172: trigger ring; 1721: guide column; 173: fitting slope; 174: elastic arm; 175: contact member; 18: mounting port; 19: clamping slot;
2: bottom housing; 21: connection section; 22: supporting section; 23: notch; 24: clamping convex rib; 25: clearance slot;
3: unlocking member; 31: manipulation portion; 32: trigger portion; 321: mounting slot; 33: connection rib;
4: driving unit; 41: boosting spring; 42: driving member; 421: elastic rib; 422: stop portion; 423: guide slope; 424: guide slot; 43: transmission member; 431: stop structure; 432: needle withdrawal channel; 433: sliding chute;
5: puncture unit; 51: tension spring; 52: hub; 521: fitting structure; 53: connector; 531: fitting portion; 532: clamping structure;
6: monitoring unit; 61: housing; 611: mounting position; 612: positioning hole; 613: limiting rib; 62: adhesive member; 63: monitoring module; 631: first clamping portion; 64: electronic module;
7: sealing member; 71: first sealing portion; 711: first folding portion; 712: first operation portion; 72: second sealing portion; 721: second folding portion; 722: second operation portion;
8: sealing unit; 81: sealed chamber; 82: receiving member; 821: protrusion portion; 83: first passing opening; 84: second passing opening;
9: fixing member; and 91: opening.

### DETAILED DESCRIPTION

To more clearly describe the overall idea of this application, the following detailed description is exemplarily provided with reference to the accompanying drawings of this specification.

Many specific details have been elaborated in the following description to facilitate a full understanding of this application, but this application can also be implemented in other ways different from those described here. Therefore, the protection scope of this application will not be limited by the following specific embodiments disclosed.

In addition, in the descriptions of this application, it should be understood that orientations or positional relationships indicated by "top", "bottom", "inner", "outer", "axial", "radial", "circumferential", and the like are orientations or positional relationships as shown in the drawings, and are only for the purpose of facilitating and simplifying the descriptions of this application instead of indicating or implying that devices or elements indicated need to have particular orientations, and be constructed and operated in the particular orientations, so that these terms are not construed as limiting this application.

In this application, unless otherwise expressly specified and limited, the terms "mount", "connect", "connection", "fix", and the like should be understood in a broad sense, such as, a fixed connection, a detachable connection, an integrated connection, a mechanical connection, an electrical connection, communication, a direct connection, an indirect connection through an intermediate medium, an internal communication of two elements, or interaction between two elements. A person of ordinary skill in the art may understand the specific meanings of the foregoing terms in this application according to specific situations.

In this application, unless otherwise explicitly stipulated and restricted, that a first feature is "on" or "below" a second feature may include that the first and second features are in direct contact, or may include that the first and second features are in indirect contact using an intermediate. In the description of this specification, the description referring to the terms "implementation", "embodiment", "one embodiment", "example", "specific example", or the like means that specific features, structures, materials or characteristics described in connection with the embodiments or examples are included in at least one embodiment or example of this application. In this specification, the schematic representations of the above terms are not necessarily intended to refer to the same embodiment or example. Furthermore, the specific features, structures, materials or characteristics described may be combined in any suitable manner in any one or more embodiments or examples.

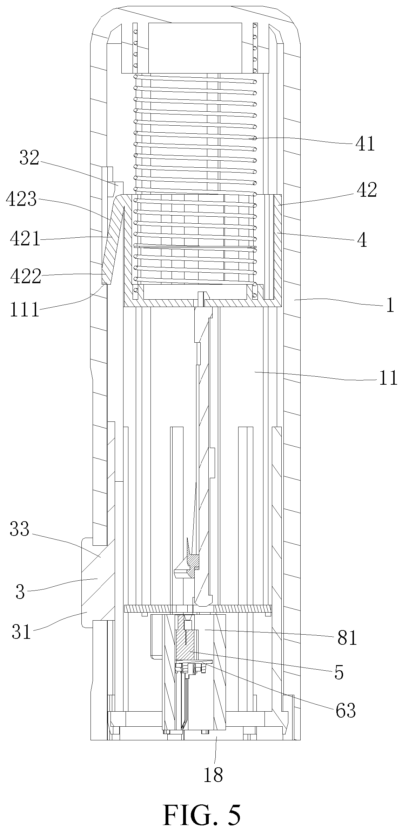

As shown in FIG. 5 to FIG. 7, an implantation device for implanting an analyte sensor into a subcutaneous tissue includes an outer housing 1, where the outer housing 1 has a first end and a second end that are oppositely disposed in a first direction; a mounting port 18 is formed in the first end; a mounting channel 11 extending in the first direction is disposed within the outer housing 1; a sealing unit 8 disposed within the mounting channel 11, where the sealing unit 8 has a sealed chamber 81 that is isolated from the mounting channel 11; a monitoring unit 6, where the monitoring unit 6 includes a housing 61, an electronic module 64, and a monitoring module 63; the housing 61 is detachably arranged at the first end; the electronic module 64 is disposed within the housing 61; the monitoring module 63 is disposed within the sealed chamber 81; and a driving unit 4, where the driving unit 4 is disposed within the mounting channel 11, and the driving unit 4 is capable of driving the monitoring module 63 to move in the first direction such that the monitoring module 63 is assembled to the housing 61, the monitoring module 63 is electrically connected to the electronic module 64, and the monitoring module 63 is partially implanted into a subcutaneous tissue of an implantee.

In this application, the independent sealing unit 8 is provided inside the outer housing 1, and the monitoring module 63 is mounted inside the sealing unit 8 to form an independent sterilization unit, so that a volume of the sterilization unit that requires irradiation sterilization can be reduced, and a plurality of sterilization units can be sterilized once, thereby enhancing sterilization efficiency and reducing sterilization costs. In addition, the minimization of the sterilization unit helps to minimize the entire implantation device, so that the costs can be reduced. Moreover, the sterilization unit is an independent unit, so that it is relatively convenient to assemble the sterilization unit, the assembling process can be optimized, and a degree of automation can be increased.

It should be noted that in this application, the monitoring module 63 may be mounted inside the sealing unit 8, so that the sealing unit 8 and the monitoring module 63 jointly form an independent sterilization unit. During sterilization, the sterilization unit may be mounted inside the outer housing 1 after being sterilized. Or, the sterilization unit may be first mounted inside the outer housing 1, and then the sterilization unit and the outer housing 1 are both sterilized. This is not limited here.

It should be noted that, in the initial state, the monitoring unit 6 may be entirely located within the sealing chamber 81, or may be partially located within the sealing chamber 81. Preferably, as shown in the figure, the sealing unit 8 forms, within the mouting channel 11, a sealing chamber 81 that is isolated from the mounting channel 11. In the initial state, the monitoring module 63 is arranged within the sealing chamber 81, while the housing 61 is arranged outside the sealing chamber 81, so that the monitoring module 63 is isolated from the external environment, thereby ensuring the cleanliness of the monitoring module 63.

The monitoring module 63 includes a sensor. The electronic module 64 includes a transmitter. In an initial state, the monitoring module 63 is fixed inside the outer housing 1. The electronic module 64 is fixed inside the housing 61, and the housing 61 and the outer housing 1 are in a split type. Therefore, the two modules may be separately sterilized in two different sterilization manners. For example, after the monitoring module 63 is fixed inside the outer housing 1 (in this case, the housing 61 is not mounted to the outer housing 1), radiation sterilization is performed on components inside the outer housing 1 to complete sterilization on the monitoring module 63 and other components inside the outer housing 1, and separate gas sterilization can be performed on the housing 61 and electronic units inside it. The two different sterilization manners are completed before the outer housing 1 and the housing 61 are assembled (that is, in a state in which the monitoring module 63 and the electronic module 64 are separated). Therefore, damage to other components caused by a sterilization manner can be avoided. After the sterilization is completed, the housing 61 is assembled to the outer housing 1, to complete the assembling, and subsequent packaging and delivery are performed. Since the assembling is completed before delivery, when a user gets a product, the entire product has been completely sterilized and assembled, so that the user does not need to assemble the product by self. Thus, a difficulty of usage is lowered, and convenience of use is enhanced. In addition, a battery for supplying power to the electronic module 64 may be arranged on the monitoring module 63, so that before implantation is triggered, the battery and the electronic module 64 are also in a separated state, and the battery and the electronic module 64 are not electrically connected. Therefore, a case in which the battery is discharged before use by a user can be reduced. Meanwhile, warehousing time is prolonged, and power storage of the battery is ensured. After the product is implanted by the user, the battery can have a relatively long battery life, thereby enhancing a user experience. The monitoring module 63 may be spaced apart from the housing 61 along a first direction.

The implantation device further comprises a bottom housing 2, which is detachably connected to the outer housing 1. When the bottom housing 2 is separated from the outer housing 1, the sealing chamber 81 communicates with the mounting channel 11.

In the present application, the bottom housing 2 is detachably connected to the outer housing 1. When the bottom housing 2 is separated from the outer housing 1, the sealing chamber 81 communicates with the mounting channel 11, such that the bottom housing 2 can be removed and the seal released in a single operation. This reduces the number of operational steps required from the user and improves the user experience. In addition, it can effectively prevent situations in which the user forgets to release the seal before starting implantation, thereby reducing the probability of implantation failure.

Of course, in other embodiments, the monitoring module 63 and the electronic module 64 may also be assembled together before leaving the factory and arranged within the sealing chamber 81, which is not limited herein.

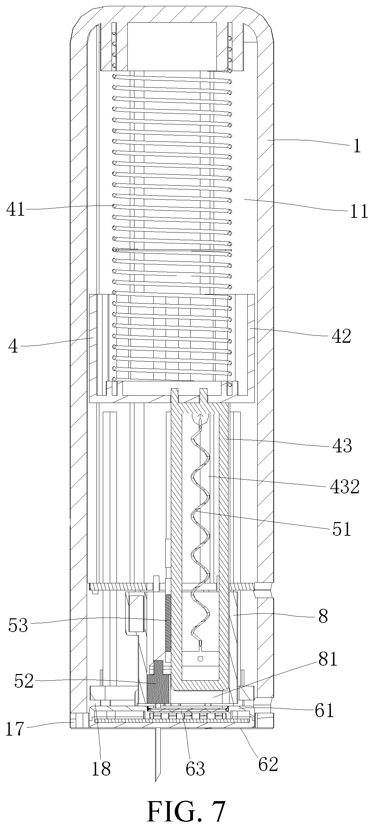

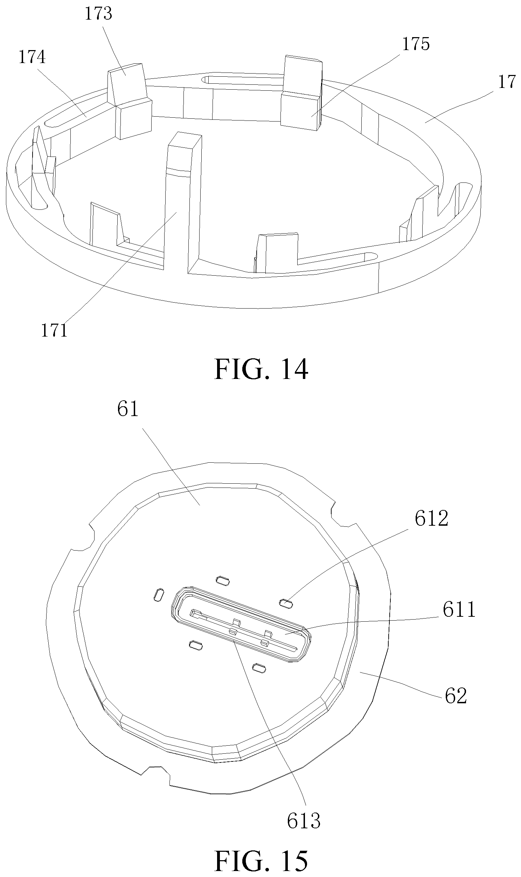

Further, as shown in FIG. 7 and FIG. 15, the housing 61 has a mounting position 611 for receiving the monitoring module 63. A trigger member is triggered to mount the monitoring module 63 into the mounting position 611 and cause a puncture unit 5 to penetrate the subcutaneous tissue.

After one trigger operation of a user, the driving unit 4 drives the puncture unit 5 and the monitoring module 63 to move in the first direction to perform an implantation action. In this process, the monitoring module 63 is mounted to the mounting position 611 of the housing 61, to complete the assembling of the monitoring module 63 and the electronic module 64 in the monitoring unit 6. In addition, a puncture assembly carrying a detection module penetrates a subcutaneous tissue of the user to complete implantation. The entire process only requires one trigger operation of the user, and an assembling operation on a detection unit does not need to be separately performed. This simplifies operation steps and enhances convenience of use, and can also prevent pollution caused by destroying of a sealed environment of the monitoring module 63 and the electronic module 64 in the self-assembling process of the user.

Specifically, as shown in FIG. 15, a shape of the mounting position 611 matches a shape of the monitoring module 63.

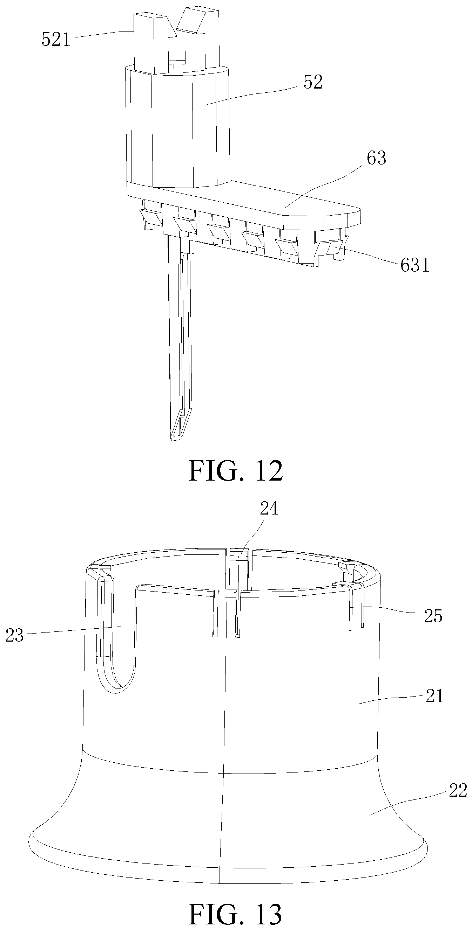

This application does not limit an assembling manner of the monitoring module 63 and the housing 61. Preferably, as shown in FIG. 12 and FIG. 15, the monitoring module 63 is provided with a first clamping portion 631, and a second clamping portion is disposed within the mounting position 611. The first clamping portion 631 and the second clamping portion are clamped and fixed.

By the clamping and fixing manner, the connection between the first clamping portion 631 and the second clamping portion is simpler and more convenient. In addition, the first clamping portion 631 and the second clamping portion can be automatically clamped under a driving force of the driving unit 4 in the first direction, so that no additional operation is required.

Specifically, one of the first clamping portion 631 and the second clamping portion may be a hook, and the other of the first clamping portion and the second clamping portion may be a hook slot, so that the hook and the hook slot are in clamping fit. Or, both the first clamping portion 631 and the second clamping portion may be configured as hooks, which can complete the clamping fit as well. Preferably, slopes or cambered surface structures are arranged on one sides, opposite to each other, of the first clamping portion 631 and the second clamping portion, so that under a relative force in an axial direction of the outer housing 1, clamping between the first clamping portion 631 and the second clamping portion can be completed.

Further, a first electrical connection portion is provided inside the mounting position 611. The monitoring module 63 is provided with a second electrical connection portion. When the monitoring module 63 is placed into the mounting position 611, the first electrical connection portion is electrically connected to the second electrical connection portion.

While the monitoring module 63 completes structural connection to the housing 61, the first electrical connection portion and the second electrical connection portion also complete coupling with each other, to implement electrical connection. Therefore, a battery for supplying power to the electronic module 64 may be arranged on the monitoring module 63, so that before implantation is triggered, the battery and the electronic module 64 are also in a separated state, and the battery and the electronic module 64 are not electrically connected. After implantation is triggered, the battery is electrically connected to the electronic module 64, to supply power to the electronic module 64.

Specifically, the first electrical connection portion is an elastic sheet, and the second electrical connection portion is an electrode. After the monitoring module 63 enters the mounting position 611, the elastic sheet is connected to two sides of the electrode, to complete the electrical connection.

Preferably, as shown in FIG. 15, a limiting rib 613 is arranged on an outer circumferential side of the mounting position 611, to limit the monitoring module 63 after the monitoring module 63 is mounted in place, so as to prevent the monitoring module 63 from being detached from the mounting position 611. As shown in FIG. 15, a plurality of positioning holes 612 are further formed in a periphery of the mounting position 611 in a spacing manner. The monitoring module 63 is provided with a plurality of positioning convex ribs corresponding to the positioning holes 612, to implement plug-in fit with the positioning holes 612 for positioning. The positioning holes 612 surround an outer side of the limiting rib 613. In addition, a sealing adhesive strip is further arranged at an edge of the mounting position 611 and is configured to seal a gap between the monitoring module 63 and the mounting position 611.

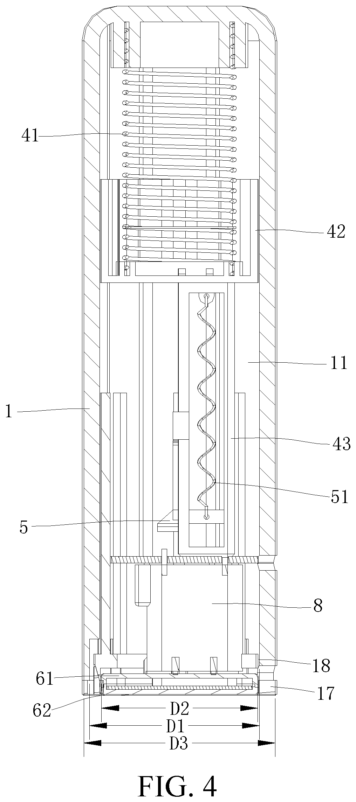

In a preferable implementation of this application, as shown in FIG. 4 to FIG. 11, a sealing unit 8 is further disposed within the mounting channel 11. Inside the mounting channel 11, the sealing unit 8 encloses a sealed chamber 81 insulated from the mounting channel 11. In an initial state, the monitoring module 63 is arranged inside the sealed chamber 81, and the housing 61 is arranged outside the sealed chamber 81, so as to isolate the monitoring module 63 from an external environment and ensure cleanness of the monitoring module 63.

In one embodiment, in an initial state, the puncture unit 5 is located outside the sealed chamber 81. After the implantation device is triggered, the puncture unit 5 moves into the sealed chamber 81 in the first direction, and drives the monitoring module 63 to move, so that the puncture unit 5 is assembled with the housing 61 and is implanted into a subcutaneous tissue of a user.

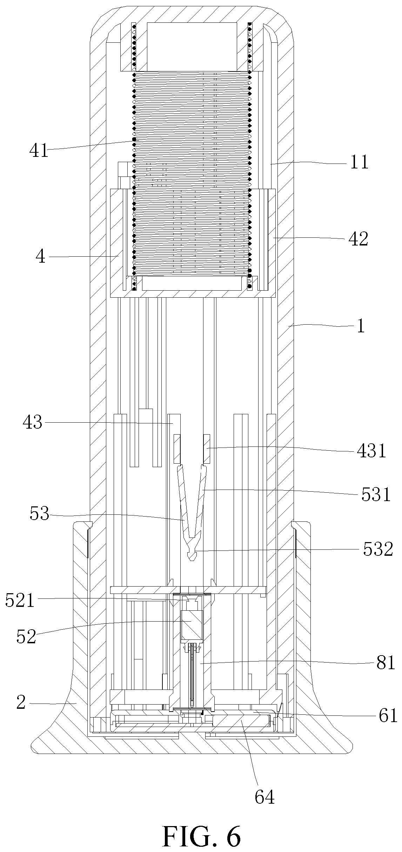

In another embodiment, as shown in FIG. 6 to FIG. 9, the puncture unit 5 includes a hub 52 having a puncture needle, and a connector 53. In an initial state, the hub 52 is located inside the sealed chamber 81, and is fixed to the monitoring module 63. A connection rib 33 is located outside the sealed chamber 81 and is separated from the sealed chamber.

After the implantation device is triggered, the driving unit 4 drives the connector 53 to move in the first direction to enter the sealed chamber 81, to complete assembling with the hub 52, and drives the monitoring module 63 to move in the first direction to be assembled with the housing 61, to complete implantation. In this case, the connector 53 and the hub 52 are assembled into a whole. After implantation is completed, the connector 53 and the hub 52 move together in a second direction to perform a needle withdrawal action.

Specifically, as shown in FIG. 7 to FIG. 9, the driving unit 4 includes a driving member 42 and a transmission member 43 that are connected to each other. The transmission member 43 internally has a needle withdrawal channel 432. The connector 53 is locked to the transmission member 43. A tension spring 51 is disposed within the needle withdrawal channel 432. In the initial state, the tension spring 51 is in a stretched state. An unlocking structure is disposed within the mounting channel 11. When the connector 53 moves to an end of a travel in first direction, the unlocking structure triggers the connector 53 to cause the connector 53 to move along the needle withdrawal channel 432 in the second direction under action of the tension spring 51.

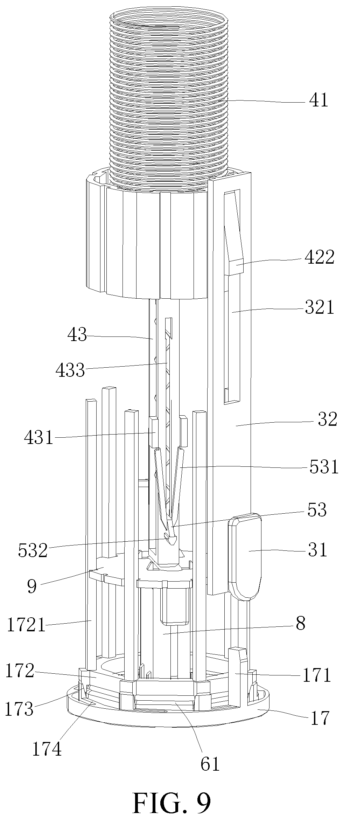

Specifically, as shown in FIG. 6 and FIG. 9, the connector 53 is provided with a clamping structure 532. The hub 52 is provided with a fitting structure 521. In the process that the connector 53 moves in the first direction, the clamping structure 532 fits with the fitting structure 521, to assemble the connector 53 with the hub 52. Specifically, as shown in FIG. 6 and FIG. 9, the clamping structure 532 is a hook slot, and the fitting structure 521 is a hook. Certainly, the two structures may be exchanged, or the two structures may be both hooks. This is not limited here.

As shown in FIG. 6 and FIG. 9, the transmission member 43 is further provided with a stop structure 431. The connector 53 is provided with a fitting portion 531. The fitting portion 531 fits with the stop structure 431 to prevent the connector 53 from moving relative to the transmission member 43. An unlocking structure is disposed within the mounting channel 11. The unlocking structure can apply a force to the fitting portion 531 or the stop structure 431 to detach them.

In one specific embodiment, as shown in FIG. 6, FIG. 7, FIG. 8, and FIG. 9, the connector 53 has a force-bearing portion located inside the needle withdrawal channel 432. The fitting portion 531 and the clamping structure 532 are both located on an outer side of the needle withdrawal channel 432. One end of the tension spring 51 is connected to the force-bearing portion. A stop convex block is arranged on an outer surface of the transmission member 43 to form the stop structure 431. The fitting portion 531 is an elastic convex rib with one end connected to the clamping structure 532. The elastic convex rib and the stop convex block fit with each other to lock the connector 53.

When the transmission member 43 carrying the connector 53 moves in the first direction to the end of the travel, the unlocking structure compresses the elastic convex rib to move, so that the elastic convex rib is misplaced from the stop convex block in the first direction, thereby completing unlocking.

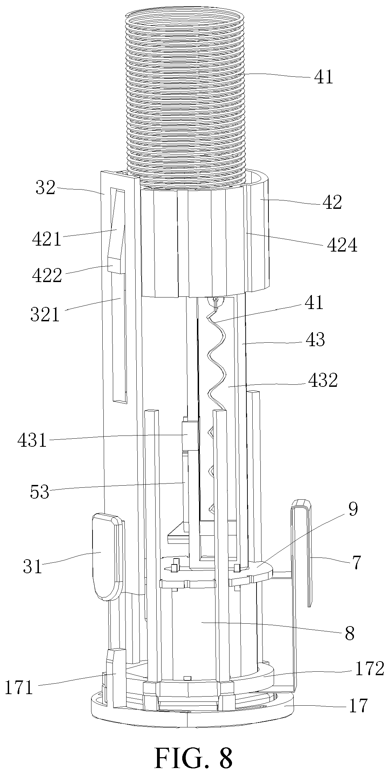

Specifically, as shown in FIG. 8 and FIG. 9, the first end is the end provided with the mounting port 18, and the second end is the fitting end 13 opposite to the first end. A fixing member 9 is disposed between the sealing unit 8 and a fitting end 13 of the outer housing 1. The fixing member 9 is of a plate-shaped structure and has an opening 91 through which the transmission member 43 and the connector 53 enter the sealed chamber 81. An edge of the opening 91 forms an unlocking structure. When the connector 53 passes through the opening 91, the edge of the opening 91 compresses the elastic convex rib to move.

Preferably, as shown in FIG. 9, a sliding chute 433 extending in the first direction is formed in an outer surface of the transmission member 43. Stop structures 431 are arranged on two sides of the sliding chute 433. The connection portions are correspondingly provided with two fitting portions 531 that are in one-to-one correspondence fit with the stop structures 431 on the two sides.

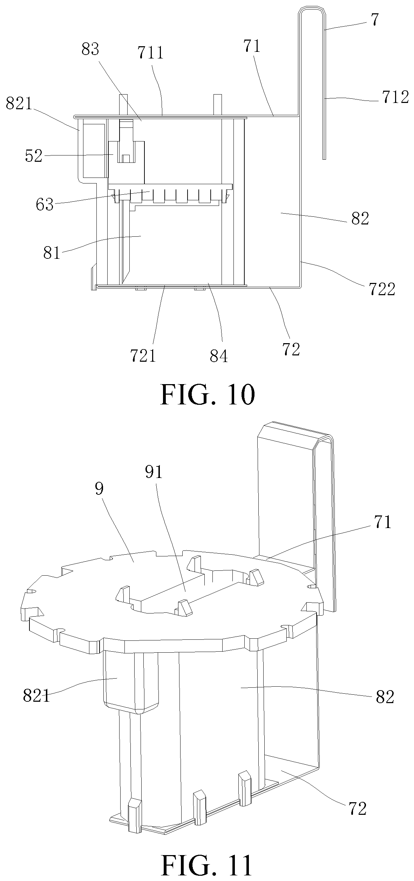

As shown in FIG. 10 and FIG. 11, the sealing unit 8 includes a receiving member 82 and a sealing member 7 detachably connected to the receiving member 82. The receiving member 82 encloses the sealed chamber 81. An upper end is fixedly connected to the fixing member 9, and a lower end is abutted with the housing 61 of the monitoring unit 6. Communication ports are respectively formed in two ends of the receiving member 82 in the first direction, and the sealing member 7 covers the communication openings, so as to sealing the sealed chamber 81. The communication openings include a first passing opening 83 located at a top end of the receiving member 82, and a second passing opening 84 located at a bottom end of the receiving member 82. The transmission member 43 and the connector 53 can enter the sealed chamber 81 through the opening 91 and the first passing opening 83.

As shown in FIG. 10 and FIG. 11, the receiving member 82 further has a protrusion portion 821 protrudes towards a transverse side, so as to form, inside the sealed chamber 81, a cavity for placing a dry piece.

Specifically, as shown in FIG. 9, a hook is arranged at a top end of the receiving member 82, and the hook is clamped and fixed to the edge of the opening 91 of the fixing member 9.

As shown in FIG. 10 and FIG. 11, the sealing member 7 further includes a first folding portion 711 and a second folding portion 721 that respectively extend from the first sealing portion 71 and the second sealing portion 72, and a first operation portion 712 and a second operation portion 722 that respectively extend from the first folding portion 711 and the second folding portion 721. One side of the first folding portion 711 facing away from the sealed chamber 81 and one side of the second folding portion 721 facing away from the sealed chamber 81 are respectively stacked on the first sealing portion 71 and the second sealing portion 72. A withdrawal port is provided in the outer housing 1. The first operation portion 712 and the second operation portion 722 extend out of the outer housing 1 from the withdrawal port.

Preferably, as shown in FIG. 10, the first sealing portion 71 and the second sealing portion 72 are both of a thin film structure, and both seal the first passing opening 83 and the second passing opening 84 through the first folding portion 711 and the second folding portion 721. The first sealing portion 71 is used an example. The first operation portion 712 is located on an outer side of the outer housing 1 in a third direction. The third direction is perpendicular to the first direction. The first sealing portion 71 covers the first passing opening 83, and one end away from the first operation portion 712 is folded downwards to form the first folding portion 711. A lowest layer of the first folding portion 711 is abutted with a top wall of the receiving member 82 to seal the first passing opening 83. When the first operation portion 712 is pulled, the first operation portion 712 drives an upper layer of the first folding portion 711 to first move in the third direction first, and the first folding portion 711 is then gradually unfolded, so that the first sealing portion 71 is detached from the receiving member 82, to open the first passing opening 83.

It should be noted that this application does not limit detachment directions of the first operation portion 712 and the second operation portion 722. The first operation portion 712 and the second operation portion 722 may move in the third direction perpendicular to the first direction to remove the first sealing portion 71 and the second sealing portion 72, or may move in the first direction or the second direction to detach the first sealing portion 71 and the second sealing portion 72. This is not limited here.

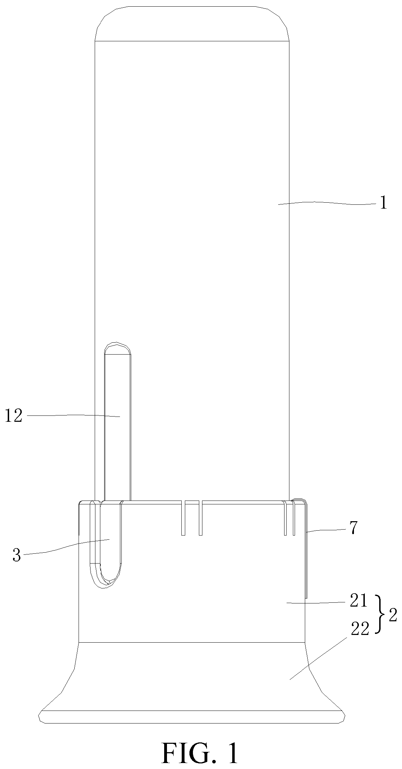

In a preferable implementation of this application, as shown in FIG. 1, FIG. 6, and FIG. 13, the implantation device further includes a bottom housing 2 detachably fixed to the mounting port 18. The bottom housing 2 closes the mounting port 18. The bottom housing 2 has a supporting portion protruding in the second direction, and the supporting portion is abutted with the adhesive member 62 to support the monitoring unit 6.

In a state in which the bottom housing 2 is not removed, the supporting portion can support the monitoring unit 6, to ensure stable connection between the monitoring unit 6 and the outer housing 1, thereby preventing the monitoring unit 6 from falling out. Preferably, an adhesive layer on a bottom surface of the adhesive member is provided with a notch 23 corresponding to the supporting portion, so as to avoid wrinkles on the adhesive member because the supporting portion rubs against glue on the adhesive layer.

The bottom housing 2 can form a relatively closed space together with the outer housing 1, and play a role in dust prevention and sealing during storage and transportation. Before use, a user first removes the bottom housing 2 to expose the mounting port 18, and further carries out implantation while one end of the outer housing 1 presses against the surface of the skin.

Preferably, the first operation portion 712 and the second operation portion 722 are fixed to the bottom housing 2, so that when removing the bottom housing 2, the user can synchronously remove the sealing member 7, and the sealed chamber 81 communicates with the mounting channel 11. Therefore, an additional operation of removing the sealing member 7 is eliminated, use steps of the user are simplified, and convenience of use is enhanced. Certainly, the sealing member 7 may be removed in another manner. For example, the user first removes the bottom housing 2 and then pulls the sealing member 7 to relieve the sealing. This is not limited here.

Preferably, as shown in FIG. 1 and FIG. 13, the bottom housing 2 includes a connection section 21 connected to the outer housing 1 and a supporting section 22 formed by extending outwards from the connection section 21 in a radial direction. An outer contour of the supporting section 22 is arc-shaped.

Before implantation, the housing 61 of the monitoring unit 6 is located inside the mounting port 18, and the adhesive member 62 is located outside the mounting port 18 and protrudes out from one end of the outer housing 1. In an axial direction of the outer housing 1, an end surface of the supporting portion can support the adhesive member 62, so that the adhesive member 62 keeps flat without wrinkles, thereby maintaining perfect and uniform glue on a surface of the adhesive member 62, and maintaining a effect and stability of adhesion with the surface of the skin of a user. In addition, since the outer housing 1 is of an elongated structure, in warehousing and transportation processes, if the outer housing 1 is placed upright, a contact area between an end surface of the outer housing 1 and a countertop is relatively small, and the outer housing 1 is unstably placed and easily topples over. If the outer housing 1 is transversely placed, since an outer circumferential surface of the outer housing 1 is similar to a cambered surface, the outer housing 1 easily rolls over. By arranging the supporting portion that expands outwards at an end of the outer housing 1, a diameter of the end of the outer housing 1 can be increased. Thus, when the outer housing 1 is placed upright, the contact area between the end surface and the countertop can be enlarged, and the outer housing 1 is placed more stably.

In addition, due to the arc-shaped contour of an outer surface of the supporting section 22, transition between the connection section 21 and the supporting section 22 is smoother. This can improve a hand feel of gripping by a user, and prevent the user from feeling abrasive because of formation of a relatively large step on the outer surface.

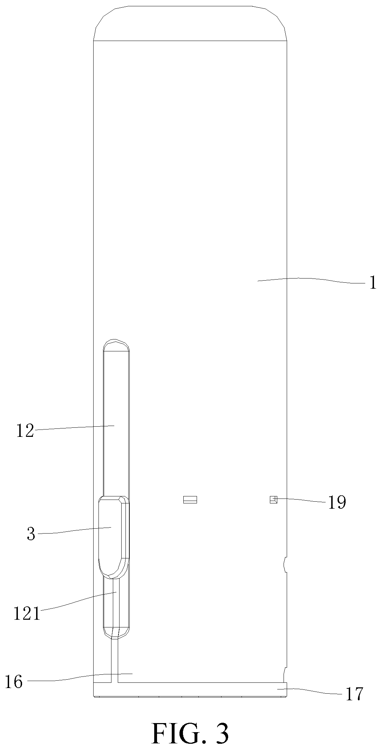

Preferably, the bottom housing 2 is sleeved on the outer side of the outer housing 1. This application does not limit a manner of assembling the bottom housing 2 with the outer housing 1. Preferably, as shown in FIG. 3 and FIG. 13, a clamping convex rib 24 is arranged on one of an outer wall of the outer housing 1 and an inner wall of the connection section 21, and a clamping slot 19 is formed in the other one of the outer wall of the outer housing 1 and the inner wall of the connection section 21. The clamping convex rib 24 fits with the clamping slot 19 to fix the bottom housing 2 to the outer housing 1.

In a specific example, as shown in FIG. 3 and FIG. 13, a plurality of clamping slots 19 are formed in a spacing manner in the outer circumferential surface of the outer housing 1 in a circumferential direction, and the connection section 21 is provided in the circumferential direction with clamping convex ribs 24 that correspond to the clamping slots 19 and protrude towards the outer housing 1. Clearance slots 25 are formed in two sides of the clamping convex ribs 24, so that the clamping convex ribs 24 can move in a radial direction of the outer housing 1, to facilitate mounting and removal of the bottom housing 2.

In this example, a user may pull the bottom housing 2 out from one end of the outer housing 1 by applying a force to the bottom housing 2 in the first direction. Certainly, in other examples, the bottom housing 2 and the outer housing 1 may be connected to each other in another manner, and may be removed in another manner. For example, threaded sections may be respectively provided on the outer wall of the outer housing 1 and the inner wall of the bottom housing 2, and the bottom housing 2 and the outer housing 1 are threadedly connected to each other. Further, to remove the bottom housing 2, the bottom housing 2 can be removed by being rotated.

As shown in FIG. 13, the bottom housing 2 is further provided with a notch 23 extending in the first direction, so as to be spliced with the outer housing 1 to form a sliding chute for allowing an unlocking member 3 to move in the first direction.

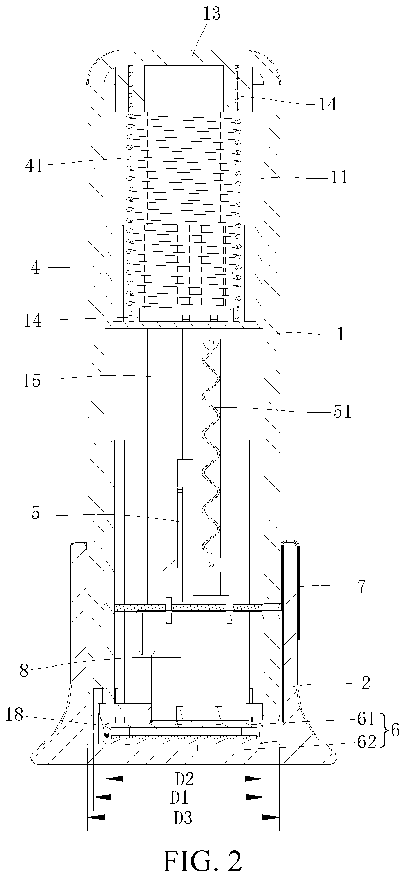

As shown in FIG. 1 and FIG. 2, the outer housing 1 internally has: a mounting channel 11 extending in a first direction, where a mounting port 18 located at an end of the outer housing 1 in the first direction is arranged at the mounting channel 11; a puncture unit 5, where the puncture unit 5 is disposed within the mounting channel 11 and can move inside the mounting channel 11; a driving unit 4, where the driving unit 4 is disposed within the mounting channel 11; the driving unit 4 is configured to be able to drive the puncture unit 5 to move in the first direction and a second direction; the second direction is opposite to the first direction; an unlocking member 3, where the unlocking member 3 is configured as a slide button arranged on one side of an outer circumferential surface of the outer housing 1; and a monitoring unit 6, where the monitoring unit 6 includes a housing 61 and a data processing component and/or a sensor disposed within the housing 61. The housing 61 is arranged at the mounting port 18.

The unlocking member 3 is a slide button with a relatively small volume and is located on one side of the outer housing 1. This not only can effectively reduce a radial size of the outer housing 1, thereby implementing a miniaturized design, but also can enable a user to complete triggering in a more comfortable posture. For example, the user may rotate the outer housing 1 to adjust an orientation of the manipulation portion 31, thereby completing the triggering with a consistently used finger and in a more comfortable angle, thereby enhancing a user experience.

In addition, the manipulation portion 31 with a relatively small volume can further effectively prevent the user from carrying out triggering mistakenly, thereby avoiding scraping of the implantation device due to a misoperation of a user.

It should be noted that the first direction is an axial direction of the outer housing 1 and is oriented towards the mounting port 18.

In the existing technology, most products are of short and thick structures, and a trigger button for triggering the implantation device is disposed on the outer circumferential surface of the outer housing 1. During use, a user needs to encirclingly grasp the outer housing 1 with a hand and then presses the trigger button to complete a trigger operation. Since the outer housing 1 has a relatively large outer diameter, it is relatively inconvenient for the user to grasp with one hand. Therefore, the user may possibly not trigger the trigger button while maintaining stability of the outer housing 1. That is, the operation cannot be completed with one hand, and as a result, the convenience of use of the product is poor.

In addition, an outer diameter of a portion of the outer housing 1 that abuts against a human skin is relatively large, and the opening 91 that is formed in the end of the outer housing 1 and through which the monitoring module 63 extends out is also relatively large. Therefore, during use of a user, the user may have a relatively great fear, leading to a poor user experience. In addition, the relatively large diameter of the outer housing 1 also makes the monitoring module 63 and a boosting assembly inside the outer housing 1 easier to shake during movement, which further exacerbates an aching feeling of the user during implantation.

As shown in FIG. 2 and FIG. 4, the monitoring unit 6 further includes an adhesive member 62 arranged on a bottom surface of the housing 61. An outer diameter D2 of the housing 61 is less than an inner diameter D1 of the mounting port 18, and the inner diameter D1 of the mounting port 18 is less than or equal to an outer diameter D3 of the adhesive member 62.

In this application, the implantation device has an initial state, a triggered state, and a retrieval state. In the initial state, partial regions of the driving unit 4, the puncture unit 5, and the monitoring unit 6 are locked inside the mounting channel 11. After a user triggers the unlocking member 3, the implantation device switches to the triggered state. In this case, the driving unit 4 is unlocked to drive the puncture unit 5 to move towards the mounting port 18 in the first direction to perform a needle insertion action. A needle head of the puncture unit 5 carrying contact pin of the sensor of the monitoring unit 6 punctures skin of the user. The sensor is implanted into a subcutaneous tissue of the user, and the adhesive member 62 of the monitoring unit 6 is adhered and fixed to the surface of the skin of the user. When the puncture unit 5 moves to an end of a travel in the first direction, the puncture unit 5 is triggered to be unlocked, so that the implantation device switches back to the retrieval state. In this state, the puncture unit 5 moves relative to the driving unit 4 in the second direction to perform a needle withdrawal action, and the needle head is withdrawn in the second direction and retracts into the outer housing 1, thus completing retrieval.

Preferably, as shown in FIG. 3, the outer housing 1 is of a structure similar to a cylinder. That is, the outer circumferential surface of the outer housing 1 is an arc surface, and a cross section of the outer housing 1 is circular. Certainly, the cross section of the outer housing 1 may be square, or elliptical, or in another shape. In this case, a diameter of the outer housing 1 and a diameter of the mounting port 18 are equivalent circular diameters corresponding to a smallest edge or a short diameter.

In this application, the monitoring unit 6 includes a housing 61 and an adhesive member 62 arranged on a bottom surface of the housing 61. A monitoring module 63, an electronic module 64, a battery, and the like are received inside the housing 61. An adhesive layer is provided on a bottom surface of the adhesive member 62 and is configured to be adhered and fixed to the surface of the skin of a user, so that after the monitoring module 63 is implanted into the subcutaneous tissue of the user, the monitoring unit 6 can be fixed to the surface of the skin of the user, so as to implement wearing. During use, one end at which the mounting port 18 is located is configured to be abutted with the surface of the skin of a user, so that the skin of the user blocks the mounting port 18. Further, during implantation, the monitoring unit 6 is provided with a force in the first direction through the driving unit 4, to move in the first direction and be adhered fixed to the skin of the user.

An outer diameter of the housing 61 is less than an inner diameter of the mounting port 18, so that before implantation, the housing 61 can be received inside the mounting port 18, that is, inside the outer housing 1, to prevent the housing 61 from protruding out of the outer housing 1, thereby protecting the housing 61, preventing the housing 61 from falling off due to collision, and reducing the entire volume. In addition, that the outer diameter of the housing 61 is less than the inner diameter of the mounting port 18 further enables the housing 61 to move inside the mounting port 18 in the first direction without interfering with the outer housing 1, thereby ensuring a smooth implantation action.

The inner diameter of the mounting port 18 is less than or equal to an outer diameter of the adhesive member 62. In one aspect, the diameter of the outer housing 1 is relatively small, so that the entire outer housing 1 is of an elongated shape, so that it is more convenient for a user to grasp the outer housing 1 during use and completely encirclingly grasp the outer housing 1. For example, the user can stably grasp the outer housing 1 with a few of fingers. Therefore, more fingers can be relieved to operate a trigger member located on an outer surface of the outer housing 1 to complete triggering. Thus, one-hand triggering can be implemented more easily without the help of another tool or another person, so that it is simpler and more convenient to use the implantation device. In another aspect, the mounting port 18 has a relatively small inner diameter, so that a hole formed in an end portion of the outer housing 1 is relatively small. This can greatly relieve a sense of fear of the user. In addition, in the implantation process, due to the relatively small port diameter of the mounting port 18, a radial space of the monitoring unit 6, the driving unit 4, and the puncture unit 5 is reduced, and a motion limiting function can be achieved, so that when moving components move in the first direction to perform an implantation action, a probability of shaking and an amplitude of shaking are smaller, thereby enhancing motion reliability and relieving an aching feeling of the user caused by shaking of a needle head.

In addition, due to the size design of the inner diameter of the mounting port 18, before implantation, the housing 61 of the monitoring unit 6 is located inside the mounting port 18, and the adhesive member 62 is located outside the mounting port 18 and protrudes out from one end of the outer housing 1. Therefore, in the axial direction of the outer housing 1, an end surface of one end of the outer housing 1, namely, an end surface on which the mounting port 18 is located, can support the adhesive member 62, so that the adhesive member 62 remains flat without wrinkles, thereby maintaining perfect and uniform glue on a surface of the adhesive member 62, and maintaining a effect and stability of adhesion with the surface of the skin of the user.

In one embodiment, the outer housing 1 has an abutment section 16 located at an end of the outer housing 1 in the first direction, and an inner wall of the abutment section 16 encloses the mounting port 18. In another embodiment, a stop rib protruding towards an inside of the mounting channel 11 is arranged on an inner wall of the abutment section 16, and the stop rib encloses the mounting port 18, so that a diameter of the mounting port 18 is less than an inner diameter of the abutment section 16.

It should be noted that in one embodiment, as shown in FIG. 4, the inner diameter of the mounting port 18 is less than the outer diameter of the adhesive member 62, so as to axially support the adhesive member 62. Preferably, the outer diameter of the adhesive member 62 is the same as or less than an outer diameter of the end at which the mounting port 18 of the outer housing 1 is located. Certainly, the inner diameter of the mounting port 18 may be the same as the outer diameter of the adhesive member 62. This is not limited here.

Preferably, a ratio of an outer diameter of the outer housing 1 to a length of the outer housing 1 in the first direction is 1: 3 to 1: 6.

An outline structure of the outer housing 1 is defined by designing the ratio of the outer diameter to the length of the outer housing 1, so that the entire outer housing 1 is of an elongated structure, thereby facilitating grasping and using by a user, and providing a better user experience.

Further, the ratio of the outer diameter of the outer housing 1 to the length of the outer housing 1 in the first direction is 1: 3.5 to 1: 4.5. Preferably, the ratio of the outer diameter of the outer housing 1 to the length of the outer housing 1 in the first direction is 1: 3.5. Preferably, the outer diameter of the outer housing 1 is 28 mm to 35 mm. The length of the outer housing 1 is 100 mm to 120 mm.

In one specific embodiment, the outer diameter of the outer housing 1 is 28 mm (about 1.1 inches), and the length of the outer housing 1 in the first direction is 98 mm (about 3.85 inches). Preferably, the implantation device further includes a bottom housing 2 detachably fixed to the end at which the mounting port 18 is located. When the bottom housing 2 is mounted on the outer housing 1, a length of the entire implantation device in the first direction is 103 mm (about 4 inches).

Preferably, as shown in FIG. 3, the outer housing 1 has an abutment section 16 located at an end of the outer housing 1 in the first direction. The mounting port 18 is located at the abutment section 16, and the abutment section 16 is configured to be abutted with the skin of an implantee. An outer diameter of the abutment section 16 is 28 mm to 35 mm.

The size design of the abutment section 16 can directly affect the port diameter of the mounting port 18 and a size of a portion of the outer housing 1 that is in direct contact with the skin of a user. By using the above size ranges, the inner diameter of the mounting port 18 can be reduced as much as possible while ensuring stability and comfort level of the abutment between the abutment section 16 and the skin of the user, thereby enhancing a guide limiting effect on the housing 61, reducing shaking of the monitoring unit 6, and relieving a sense of fear and an aching feeling of the user.

Preferably, as shown in FIG. 3, the outer diameter of the outer housing 1 in the first direction remains unchanged, that is, an outer diameter of the abutment section 16 is the outer diameter of the outer housing 1. Certainly, the outer housing 1 may be of a diameter-changing structure in the first direction, so that the outer diameter of the abutment section 16 is greater than or less than an outer diameter of another portion of the outer housing 1.

This application does not limit a driving manner of the driving unit 4, that is, an implantation manner of the implantation device. The implantation device can use either automatic implantation or manual implantation. Specifically, in one implementation, the implantation device uses a manual implantation manner. That is, the implantation device further includes an operation member. The operation member is partially located inside the mounting channel 11 and is partially located outside the outer housing 1. A user can operate the operation member from an outside of the outer housing 1 in the first direction, so that the operation member drives the driving unit 4 to move in the first direction to perform an implantation action. That is, power of the driving unit 4 is provided by the user. A speed in an entire implantation process is manually controlled by the user. That is, when the user stops pushing the operation member, the driving unit 4 also stops moving.

In a preferable implementation of this application, the implantation device uses an automatic implantation manner. As shown in FIG. 2 and FIG. 4, the driving unit 4 includes a driving member 42. The mounting channel 11 has a fitting end 13 facing away from the mounting port 18 in the first direction. A boosting spring 41 is disposed between the fitting end 13 and the driving member 42. A limiting slot 14 is formed in the fitting end 13 and/or the driving member 42, and an end portion of the boosting spring 41 is disposed within the limiting slot 14.

Power of the driving unit 4 is provided by the boosting spring 41. In an initial state, the driving member 42 is locked inside the mounting channel 11. In this case, the boosting spring 41 is in a compressed state. After a user unlocks the driving unit 4, the boosting spring 41 pushes the driving member 42 under an elastic force of the boosting spring 41, so that the driving member 42 moves in the first direction to perform an implantation action. This manner implements automatic implantation. In addition, using the boosting spring 41 for driving can better control a moving speed of the driving member 42, so that the driving member 42 tends to approach a constant speed. Moreover, a moving direction is more stable, thereby avoiding jamming in a moving process, reducing shaking, and relieving an aching feeling of a user in an implantation process.

The limiting slot 14 can limit an end of the boosting spring 41, so that the boosting spring 41 always deforms in its axial direction, and provides a stable force in the first direction for the driving member 42. Specifically, as shown in FIG. 2 and FIG. 4, the first end is one end provided with the mounting port 18, and the second end is the fitting end 13 opposite to the first end. A limiting slot 14 is formed in one side of the driving member 42 facing the fitting end 13. Meanwhile, a limiting slot 14 is also formed in an inner wall of the fitting end 13, so that two ends of the boosting spring 41 are located inside the limiting slots 14. As shown in FIG. 2 and FIG. 4, an enclosure wall protruding towards the fitting end 13 is further arranged at a peripheral edge of the driving member 42, so as to limit the boosting spring 41 when a middle portion of the boosting spring 41 undergoes deformation deviating from an axis. Preferably, as shown in FIG. 4, an axis of the boosting spring 41 overlaps an axis of the outer housing 1.

As shown in FIG. 2 and FIG. 8, guide protrusion strips 15 are provided on an inner wall of the outer housing 1. The guide protrusion strips 15 extend in the first direction. Guide slots 424 that fit with the guide protrusion strips 15 are formed in an outer circumferential side of the driving unit 4, to play a role in guiding the motion of the driving unit 4, so that the motion of the driving unit 4 is more stable. The guide protrusion strips 15 are spaced apart in a circumferential direction of the outer housing 1.

Certainly, the guide slots 424 may be formed in the inner wall of the outer housing 1, and correspondingly, the guide protrusion strips 15 are arranged on the outer wall of the driving unit 4. This is not limited here.

As shown in FIG. 1, FIG. 3, and FIG. 5, the implantation device further includes an unlocking member 3. The unlocking member 3 is a slide button, is disposed on one side of the outer circumferential surface of the outer housing 1, and can move in the first direction or the second direction to unlock the driving unit 4.

It can be understood that the outer housing 1 is of a cylindrical structure extending in the first direction. Two ends of the outer housing 1 in the first direction are end surfaces of the outer housing 1, and a wall surface surrounding the first direction is the outer circumferential surface of the outer housing 1.

Motion of the trigger member in the first direction or the second direction unlocks the driving unit 4. Compared with a manner of pressing a trigger button radially inwards along the outer housing 1, a user grasps the outer housing 1 with a hand, a force application direction of a finger is inward in a radial direction of the outer housing 1, and a force application direction in which the unlocking member 3 is unlocked in an axial direction of the outer housing 1. The force application directions of the two actions are perpendicular to each other, so that a probability that the user accidentally touches the trigger member when grasping the outer housing 1 is greatly reduced, a risk that the implantation device is accidentally triggered is lowered, the implantation device can be effectively triggered and implanted, and enhancing a user experience.

Further, as shown in FIG. 3, FIG. 5, FIG. 8, and FIG. 9, the unlocking member 3 includes a manipulation portion 31 located outside the outer housing 1, a trigger portion 32 located inside the mounting channel 11, and a connection rib 33 connected between the trigger portion 32 and the manipulation portion 31. A clearance slot 121 through which the connection rib 33 passes is formed in the outer housing 1. The clearance slot 121 extends in the first direction.

The manipulation portion 31 of the unlocking member 3 is located outside the outer housing 1 for being operated by a user. The trigger portion 32 is located inside the mounting channel 11 and is configured to perform an unlocking action on the driving unit 4. The clearance slot 121 for allowing the connection rib 33 to pass through is formed in the outer circumferential surface of the outer housing 1. This can limit the connection rib 33 to limit the entire unlocking member 3, so that the unlocking member 3 can extend only in the first direction or the second direction. Moreover, a length of the clearance slot 121 constitutes a motion travel of the unlocking member 3. After the unlocking member 3 moves until the connection rib 33 is stopped by an end portion of the clearance slot 121, the unlocking member 3 cannot move in the same direction, so that the motion of the unlocking member 3 is more reliable, and the unlocking member 3 is prevented from falling out of the outer housing 1.

Preferably, as shown in FIG. 1 and FIG. 3, the manipulation portion 31 is a slide button disposed on one side of the outer circumferential surface of the outer housing 1. A receiving slot 12 extending in the first direction is formed in an outer surface of the outer housing 1. The slide button is located inside the receiving slot 12.

The manipulation portion 31 is a slide button with a relatively small volume and is located on one side of the outer housing 1. This can enable a user to complete triggering in a more comfortable posture. For example, the user may rotate the outer housing 1 to adjust an orientation of the manipulation portion 31, thereby completing the triggering with a consistently used finger and in a more comfortable angle, thereby enhancing a user experience. In addition, the manipulation portion 31 with the relatively small volume can further effectively prevent the user from carrying out triggering mistakenly, thereby avoiding scraping of the implantation device due to a misoperation of a user. Moreover, the receiving slot 12 extending in the first direction is further formed in a surface of the outer housing 1, and the manipulation portion 31 is received in the receiving slot 12. The receiving slot 12 can play a role in guiding the motion of the manipulation portion 31, making the manipulation portion 31 move more smoothly, thereby reducing jamming occurring in the motion process and relieving an aching feeling in an implantation process. Meanwhile, the manipulation portion 31 is located inside the receiving slot 12, so that an outer surface of the manipulation portion 31 does not excessively protrude out from the surface of the outer housing 1, and can even be flush with the outer circumferential surface of the outer housing 1 or be recessed relative to the outer circumferential surface of the outer housing 1, thereby further enhancing an accidental touch prevention effect.

In a preferred embodiment, as shown in FIG. 5, the driving unit 4 includes a stop portion 422. The outer housing 1 includes a stopper 111 fitting with the stop portion 422. The stop portion 422 fits with the stopper 111 to prevent the driving unit 4 from moving relative to the outer housing 1. The trigger portion 32 can extend from the clearance slot 121 into the mounting channel 11 and be abutted with the stop portion 422 or the stopper 111 to detach them.

In one specific example, a groove structure is arranged on an inner side wall of the mounting channel 11 to form the stopper 111. The stop portion 422 is a convex rib that transversely protrudes outwards from an outer circumferential side of the driving unit 4. The convex rib is located inside the groove structure to implement stop fit. The trigger portion 32 can press the stop portion 422 inwards, so that the stop portion 422 deforms or moves inwards, to detach the convex rib from the groove structure, thereby unlocking the driving unit 4.

Certainly, a protrusion structure may be arranged on an inner wall of the mounting channel 11 to form the stopper 111. This can complete stopping and locking with the stop portion 422 as well. Similarly, the trigger portion 32 may press the stopper 111 to deform or move, so as to implement detachment from the stop portion 422.

Specifically, the stop portion 422 is an elastic rib 421. A structure of the stop portion 422 is not limited in this embodiment. The stop portion 422 may extend in the first direction or the second direction, and a convex rib that transversely protrudes out to fit with the stopper 111 is arranged on an outer side of the stop portion 422. In another example, as shown in FIG. 5, FIG. 8, and FIG. 9, the stop portion 422 is an elastic rib 421 that extends obliquely, and a gap exists between an end portion of the stop portion 422 and the driving unit 4, to provide a motion space for the stop portion 422. When the trigger portion 32 presses the stop portion 422, the stop portion 422 swings inwards.

Further, as shown in FIG. 5, FIG. 8, and FIG. 9, the trigger portion 32 has a mounting slot 321. The stop portion 422 passes through the mounting slot 321 to be in abutment fit with the stopper 111. The mounting slot 321 and/or the stop portion 422 are/is provided with a guide slope 423. The trigger portion 32 drives the stop portion 422 through the guide slope 423, so that the stop portion 422 is detached from the stopper 111.

The stop portion 422 of the driving unit 4 outwards passes through the mounting slot 321 of the trigger portion 32, and fits with the stopper 111 on the inner wall of the outer housing 1 to form a stop, so that the driving unit 4 is locked, and the driving unit 4 cannot move relative to the outer housing 1. When a user drives the unlocking member 3 to move in the first direction or the second direction, the mounting slot 321 and the stop portion 422 are in contact through the guide slope 423. The guide slope 423 converts part of a vertical force of the trigger portion 32 into a transverse force, so as to form a pressure perpendicular to the first direction on the stop portion 422. Therefore, the stop portion 422 moves to release the stopping of the stopper 111, and the driving unit 4 is unlocked and starts to perform an implantation action.

In one embodiment, as shown in FIG. 5, the stop portion 422 is an elastic rib 421 that extends obliquely, so that a guide slope 423 is naturally formed on a surface of the stop portion 422. Certainly, the guide slope 423 may be disposed within the mounting slot 321. This is not limited here.

As shown in FIG. 5, FIG. 8, and FIG. 9, the unlocking member 3 moves in the first direction to trigger the driving unit 4. Therefore, the stop portion 422 extends obliquely downwards, to form a guide slope 423 on an upper surface of the stop portion 422. When the trigger portion 32 moves downwards in the first direction, a lower end of the stop portion 422 is pressed to swing inwards. Similarly, when the unlocking member 3 is designed to move in the second direction to trigger the driving unit 4, the stop portion 422 may be correspondingly configured to extend obliquely upwards, so as to form a guide slope 423 on a lower surface of the stop portion 422.

In a preferable implementation of this application, as shown in FIG. 4, the driving unit 4 includes a driving member 42 and a transmission member 43. A boosting spring 41 is disposed between the driving member 42 and the outer housing 1. The transmission member 43 is located on one side of the driving member 42 in the first direction and is close to the mounting port 18. The transmission member 43 and the driving member 42 are fixed through a fastener or in another manner to synchronously move with the driving member 42.

In a preferable implementation of this application, the outer housing 1 is provided with an elastic arm 174 extending into the mounting port 18 and a fixed portion arranged at an end of the elastic arm 174. The fixed portion is abutted with an outer circumferential surface of the housing 61.

The elastic arm 174 clamps the housing 61 inwards from an outer circumferential side of the housing 61 to fix the housing 61. It should be noted that an arrangement position of the elastic arm 174 is not limited in this application. In one embodiment, the elastic arm 174 is arranged on an inner wall of the mounting channel 11, that is, the elastic arm 174 and the outer housing 1 are of an integral structure. In another embodiment, as shown in FIG. 4, FIG. 7, FIG. 8, FIG. 9, and FIG. 14, the implantation device further includes a fixing ring 17 located at one end of the outer housing 1. An inner diameter of the fixing ring 17 is the same as an inner diameter of the abutment section 16 of the outer housing 1, and the elastic arm 174 is arranged on the fixing ring 17.

Specifically, as shown in FIG. 14, one end of the elastic arm 174 is fixed to the fixing ring 17, and another end extends in a circumferential direction of the housing 61 and has a deformation gap from the fixing ring 17. The end is in contact with the housing 61 and clamps the housing 61, so that the elastic arm 174 has a better elastic deformation capability.

Preferably, as shown in FIG. 14, a contact member 175 is arranged on one side of the fixed portion facing the housing 61. The contact member 175 is made of a flexible material and is elastically abutted with the housing 61.

The fixed portion clamps the housing 61 through the contact member 175. The contact member 175 is made of the flexible material. In one aspect, the fixed portion is in contact with the housing 61 more gently, thereby avoiding wear caused by rigid contact between the fixed portion and the housing 61. In another aspect, under a pressure, the contact member 175 may elastically deform, thereby increasing a friction force between the contact member 175 and the housing 61 to enhance fixing stability.

Preferably, the contact member 175 is made of a silica gel material. Certainly, the contact member 175 may alternatively be made of another flexible material such as felt or cotton.

Preferably, as shown in FIG. 4, FIG. 9, and FIG. 14, the fixing ring 17 is located at an end portion of the outer housing 1. The fixing ring 17 is provided with a positioning rib 171 protruding towards the outer housing 1. The positioning rib 171 extends in the second direction. The outer housing 1 has a plugging slot that fits with the positioning rib 171, so as to plug and position the fixing ring 17 with the outer housing 1.

In a preferable implementation of this application, as shown in FIG. 9, a trigger ring 172 is further disposed within the outer housing 1. At least one of the trigger ring 172 and the elastic arm 174 is provided with a fitting slope 173, so that the trigger ring 172 is in contact with the elastic arm 174 in the first direction through the fitting slope 173. The trigger ring 172 can be driven by the driving unit 4 to move in the first direction. A driving force generates, through the fitting slope 173, a component force perpendicular to the first direction, and pushes the elastic arm 174 to expand outwards to release the housing 61, thereby completing release of the monitoring unit 6.

As shown in FIG. 9, the trigger ring 172 further has a guide column 1721 extending in the first direction. A fixing member 9 is provided between the sealing unit 8 and the fitting end 13 of the housing 61. The fixing member 9 has an opening through which the guide column 1721 passes, to form a motion guidance for the trigger ring 172 in the first direction.

Wherever the details are not mentioned in this application, they can be achieved by using or drawing upon the existing technology.

The embodiments in this specification are all described progressively, and the same similar parts between the various embodiments can be referred to each other. Each embodiment focuses on differences from other embodiments.

## Claims

1. An implantation device for implanting an analyte sensor into a subcutaneous tissue, comprising:
an outer housing, wherein the outer housing has a first end and a second end that are oppositely disposed in a first direction; a mounting port is formed in the first end, and a mounting channel extending in the first direction is provided inside the outer housing;
a sealing unit disposed within the mounting channel, wherein the sealing unit has a sealed chamber, wherein the sealing chamber and the mounting channel are isolated from each other;
a monitoring unit, wherein the monitoring unit comprises a housing, an electronic module, and a monitoring module; the housing is detachably arranged at the first end; the electronic module is disposed within the housing; the monitoring module is disposed within the sealed chamber; and
a driving unit, wherein the driving unit is disposed within the mounting channel; the driving unit is capable of driving the monitoring module to move in the first direction such that the monitoring module is assembled to the housing, the monitoring module is electrically connected to the electronic module, and the monitoring module is partially implanted into a subcutaneous tissue of an implantee; optionally the driving unit is located outside the sealed chamber, and the driving unit is capable of entering the sealed chamber in the first direction to push the monitoring module to move.

2. The implantation device according to claim 1, wherein the sealing unit is provided with communication openings respectively in two ends in the first direction, and the communication openings are configured to communicate the sealed chamber with the mounting channel.

3. The implantation device according to claim 2, wherein the sealing unit comprises a receiving member and a sealing member detachably connected to the receiving member; the receiving member and the sealing member enclose the sealed chamber; the communication openings comprise a first communication opening and a second communication opening that are located at two opposite ends of the receiving member; the sealing member comprises a first sealing portion and a second sealing portion; the first sealing portion seals the first communication opening; and the second sealing portion seals the second communication opening; optionally the sealing member further comprises a first folding portion and a second folding portion that respectively extend from the first sealing portion and the second sealing portion, and a first operation portion and a second operation portion that respectively extend from the first folding portion and the second folding portion; one side of the first folding portion facing away from the sealed chamber and one side of the second folding portion facing away from the sealed chamber are respectively stacked on the first sealing portion and the second sealing portion; a withdrawal port is provided in the outer housing; and the first operation portion and the second operation portion extend out of the outer housing from the withdrawal port.

4. The implantation device according to claim 2, wherein a fixing member is further disposed within the outer housing; the fixing member is located between the sealing unit and the second end; the sealing unit is fixedly connected to the fixing member; the fixing member has an opening corresponding to the communication openings; and the driving unit is disposed between the fixing member and the second end; optionally the sealing unit is provided with a clamping hook, and the clamping hook passes through the opening and is clamped and fixed to the fixing member.

5. The implantation device according to any one of claims 1 - 4, wherein the housing has a mounting position for accommodating the monitoring module; the monitoring module is capable of moving in the first direction under driving of the driving unit, so as to be mounted in the mounting position; and a puncture unit penetrates the subcutaneous tissue; optionally the monitoring module is provided with a first clamping portion; a second clamping portion is disposed within the mounting position; the first clamping portion and the second clamping portion are clamped and fixed; a first electrical connection portion is provided inside the mounting position; the monitoring module is provided with a second electrical connection portion; and when the monitoring module is placed into the mounting position, the first electrical connection portion is electrically connected to the second electrical connection portion.

6. The implantation device according to any one of claims 1 - 5, wherein the outer housing is provided with an elastic arm extending into the mounting port and a fixed portion arranged at an end of the elastic arm; and the fixed portion is abutted with an outer circumferential surface of the housing; optionally a contact member is arranged on one side of the fixed portion facing the housing; and the contact member is made of a flexible material and is elastically abutted with the housing.

7. The implantation device according to any one of claims 1 - 6, wherein the implantation device further comprises a bottom housing, wherein the bottom housing is detachably connected to the outer housing, wherein the sealed chamber communicates with the mounting channel when the bottom housing is separated from the outer housing.

8. The implantation device according to claim 7, wherein the implantation device further comprises a puncture unit; the puncture unit comprises a hub and a puncture needle connected to the hub; the driving unit comprises a driving member and a connector connected between the driving member and the hub; at an initial position, the hub is located inside the sealed chamber and is fixedly connected to the monitoring unit; and the connector is located outside the sealed chamber; optionally the connector is provided with a clamping structure; the hub is provided with a fitting structure; and the connector is able to move in the first direction to connect the clamping structure to the fitting structure.

9. The implantation device according to claim 8, wherein the driving unit is provided with a stop structure; the puncture unit is provided with a fitting portion; the fitting portion fits with the stop structure to prevent the puncture unit from moving relative to the driving unit; an unlocking structure is disposed within the mounting channel; and the unlocking structure is able to apply a force to the fitting portion or the stop structure to detach the fitting portion from the stop structure.

10. The implantation device according to any one of the claims 7 - 9, wherein the monitoring unit comprises an adhesive member arranged on a bottom surface of the housing, and an isolation membrane separable from the adhesive member; and when the bottom housing is separated from the outer housing, the isolation membrane is separated from the adhesive member; optionally an outer diameter of the housing is less than an inner diameter of the mounting port, and the inner diameter of the mounting port is less than or equal to an outer diameter of the adhesive member.

11. The implantation device according to any one of the claims 1 - 10, wherein a ratio of an outer diameter of the outer housing to a length of the outer housing in the first direction is 1: 3 to 1: 6, optionally the outer housing has an abutment section located at an end of the outer housing in the first direction; the mounting port is located at the abutment section; the abutment section is configured to be abutted with the skin of an implantee; and an outer diameter of the abutment section is 28 mm to 35 mm; optionally an outer diameter of the outer housing is 28 mm to 35 mm; and optionally a length of the outer housing is 100 mm to 120 mm.

12. The implantation device according to any one of the claims 1 - 11, wherein the implantation device further comprises an unlocking member, wherein the unlocking member is configured as a slide button arranged on one side of an outer circumferential surface of the outer housing; and wherein the unlocking member is able to move in the first direction or the second direction to unlock the driving unit.

13. The implantation device according to claim 12, wherein the unlocking member comprises a manipulation portion located outside the outer housing, a trigger portion located inside the mounting channel, and a connection rib connected between the trigger portion and the manipulation portion; the manipulation portion is configured as the slide button; an avoidance slot through which the connection rib passes is formed in the outer housing; and the avoidance slot extends in the first direction; optionally the driving unit comprises a stop portion; the outer housing comprises a stopper fitting with the stop portion; the stop portion fits with the stopper to prevent the driving unit from moving relative to the outer housing; and the trigger portion is able to extend from the avoidance slot into the mounting channel and be abutted with the stop portion or the stopper to detach the stop portion from the stopper.

14. The implantation device according to claim 12, wherein a receiving slot extending in the first direction is formed in an outer surface of the outer housing; and the slide button is located inside the receiving slot.

15. The implantation device according to any one of claims 7 - 14, wherein the bottom housing has a supporting portion protruding in the second direction; and the supporting portion is abutted with a bottom surface of the monitoring unit to support the monitoring unit; optionally the bottom housing comprises a connection section connected to the outer housing and a supporting section formed by extending outwards from the connection section in a radial direction; and an outer contour of the supporting section is arc-shaped; and optionally a clamping convex rib is arranged on one of an outer wall of the outer housing and an inner wall of the connection section, and a clamping slot is formed in the other one of the outer wall of the outer housing and the inner wall of the connection section; and the clamping convex rib fits with the clamping slot to fix the bottom housing to the outer housing.
